# EUROPEAN PATENT APPLICATION

(11) **EP 0 922 763 A1**
(43) Date of publication of application: **16.06.1999**
(21) Application number: 97403017.3
(22) Date of filing: 12.12.1997
(51) Int. Cl.: C12N 15/12, C12N 5/10, C12N 1/21, C07K 14/705, C07K 16/18, C12Q 1/68, C12P 21/02, A61K 38/17

(54) **Inwardly rectifying potassium channel gene and protein**

(71) Applicant: SYNTHELABO, 92350 Le Plessis Robinson (FR)
(72) Inventor: Partiseti, Michel, 92160 Antony (FR); Collura, Vincent, 92290 Chatenay-Malabry (FR); Agnel, Magali, 92500 Rueil Malmaison (FR); Culouscou, Jean-Michel, 78110 Le Vesinet (FR); Graham, David, 78380 Bougival (FR)
(74) Representative: Thouret-Lemaitre, Elisabeth

(57) **Abstract**

Kir7.1 polypeptides and polynucleotides and methods for producing such polypeptides by recombinant techniques are disclosed. Also disclosed are methods for utilizing Kir7.1 polypeptides and polynucleotides in the design of protocols for the treatment of certain diseases and diagnostic assays for such conditions.

## Description

### FIELD OF INVENTION

This invention relates to newly identified polynucleotides, polypeptides encoded by them and to the use of such polynucleotides and polypeptides, and to their production. More particularly, the present invention relates to polypeptides which are inwardly rectifying potassium channel receptors, hereinafter referred to as Kir7.1.

### BACKGROUND OF THE INVENTION

Inward rectifying potassium channels (Kir) are a family of two transmembrane domain polypeptides that upon homomeric or multimeric assembly form functional potassium channels (for review see: Nichols C. G. and Lopatin A. N. (1997) *Ann. Rev. Physiol* 59: 171-191; Jan L. Y. and Jan Y. N. (1997) *Ann. Rev. Neurosci*. 20: 91-123; Doupnik C. A., Davidson N. ; Lester H. A. (1995) *Cur. Op. Neurobiol*. 5 pp 268-277 and Wo 95/21943). Members of this family have been described as potassium channels in numerous organisms from yeast to man, and classified in six different subfamilies (Kir1.0 to Kir6.0) according to the nomenclature of Chandy and Gutman (1993, *Trends Pharmacol*. *Sci*. 14: 434). Inward rectification means that at any given driving force (membrane voltage), the inward flow of K⁺ ions is greater than the outward flow for the opposite driving force.This type of channel first described in muscle cells is also expressed in many others cell types such as glial cells, neurons, myeloid cells, and pancreatic beta cells, and the major role of this channel is probably regulation of the resting membrane potential. Many of these channels are strongly dependent upon activation by hormones and transmitters, often through G proteins or other second messenger systems.

The Kir6.0 subfamily is inhibited by sulphonylureas drugs that are used in the treatment of non insulino-dependent diabete melitus (NIDDM) (Ashcroft and Rorsman, 1991, Prog. Biophys. Molec. Biol. 54, pp87-143). Agonists or antagonists of inwardly rectifying potassium channels may be used, for example, to treat cerebral and cardiac and renal ischemias, brain and cardiac diseases, auto-immune diseases and inflammation, pain, cell proliferation disorders such as cancer, to mimic or antagonize effect of endogenous neurotransmitters and hormones, to decrease or increase endogenous neurotransmitters and hormones releases, or to diagnose or treat any disorder related to abnormal expression of this protein, more particularly in brain and kidneys.

### SUMMARY OF THE INVENTION

The present applicant has found a new class of potassium channel protein. This new subunit may be implicated in different physiological processes such as neurotransmitters and hormones release, the buffering by glial cells of external K⁺ concentrations as well as cell proliferation, and it may provide a target in various pathologies.

In accordance with one aspect of the present invention, there is provided a novel mature polypeptide which is a potassium channel, as well as fragments, analogs or derivatives. The polypeptide of the present invention is of human origin and it corresponds to a novel member of the Kir family and to which we have given the designation Kir 7.1 . In accordance with another aspect of the present invention, there are provided polynucleotides (DNA) which encode such polypeptide.

In accordance with yet a further aspect of the present invention, there is provided a process for producing such polypeptide by recombinant techniques.

These and other aspects of the present invention should be apparent to those skilled in the art from the teachings herein.

### Brief Description of the Figures

The following drawings are illustrative of the embodiments of the invention and are not meant to limit the scope of the invention as encompassed by the claims.

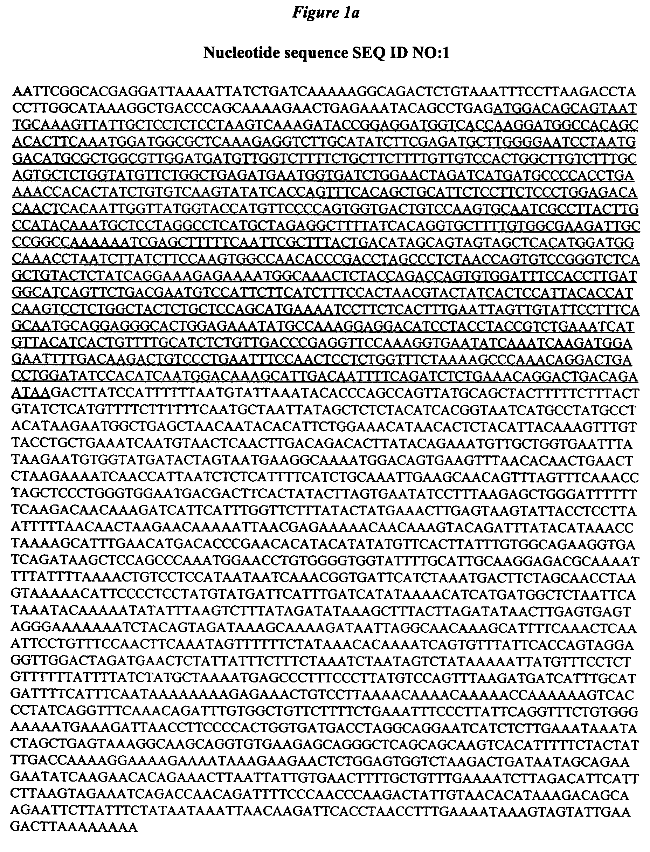

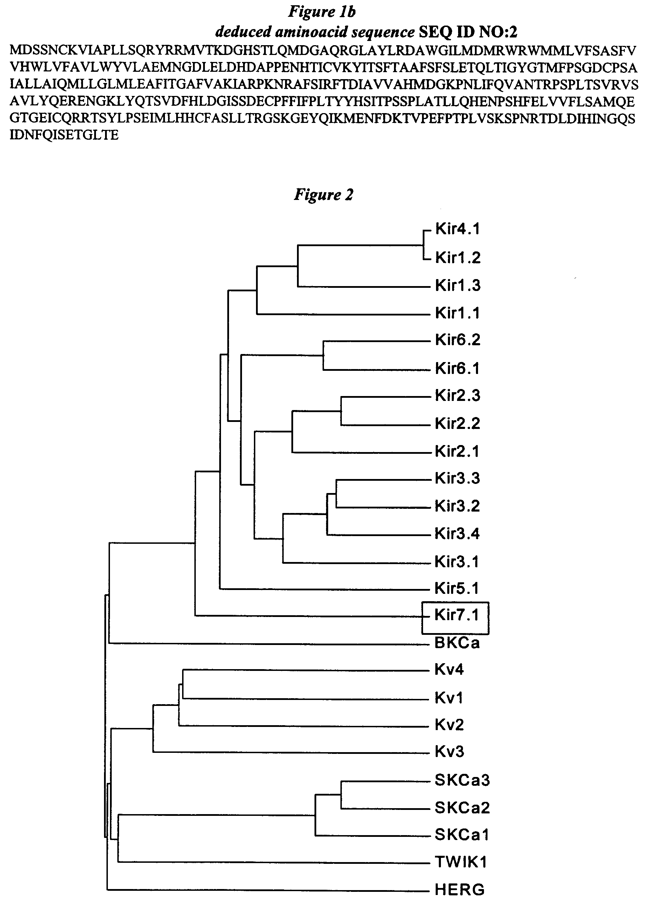

***Figure 1*** shows the cDNA (Fig.1a) and the corresponding deduced amino-acid sequence (Fig.1b) of Kir7.1 channel. The full length protein is 360 amino acid residues with a putative molecular weight of 40541 Daltons.
***Figure 2*** illustrates the phylogenic relationship between Kir7.1 channel and others members of the potassium channel superfamily.

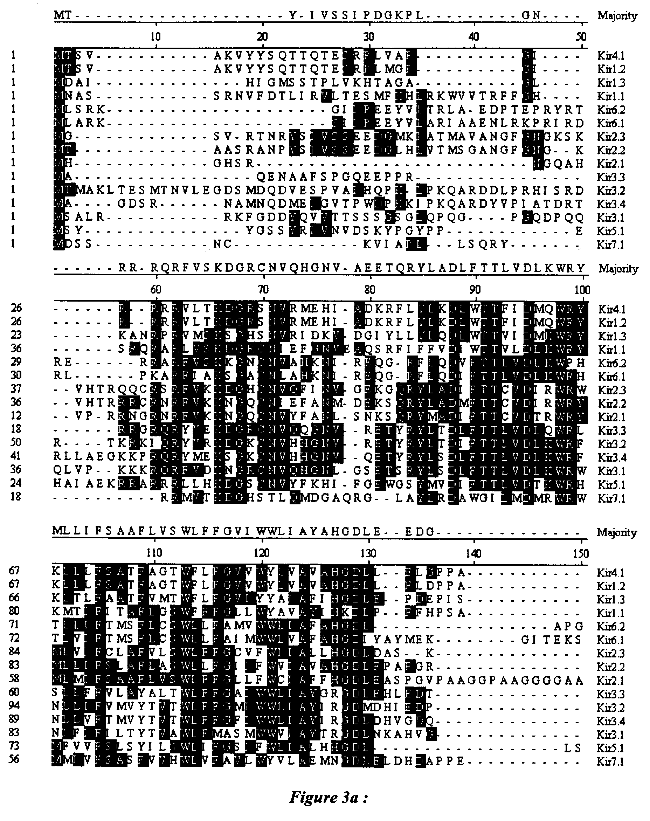

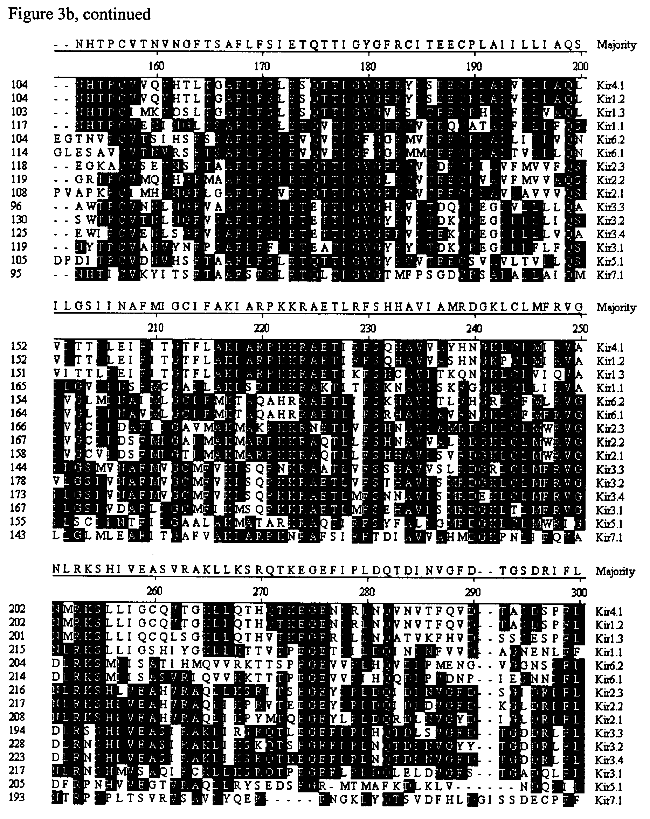

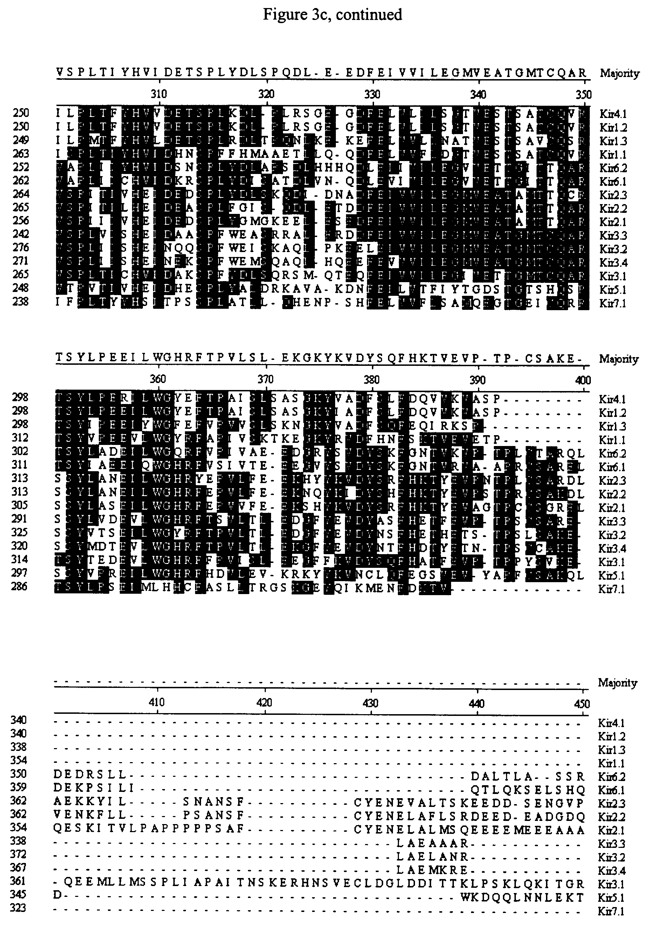

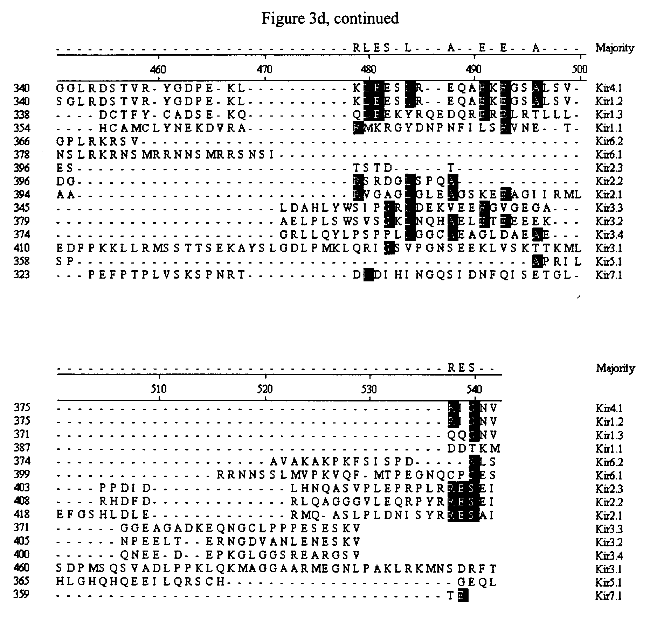

***Figure 3*** shows an aminoacid sequence comparison of the Kir7.1 channel with others members of the inwardly rectifying potassium channel family from the phylogenic tree of Figure 2. Alignment was performed with the clustal algorythm of MEGALIGN (version 3.10a ) from DNASTAR. Regions of homology are shaded in black.

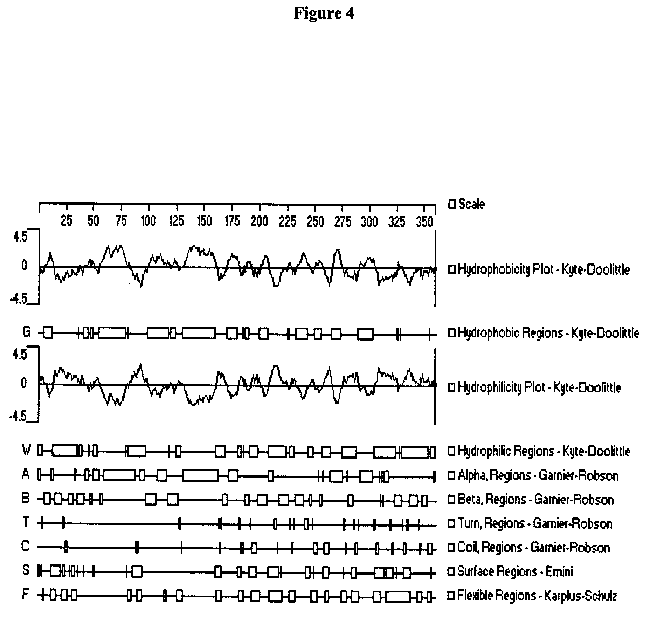

***Figure 4*** : secondary structural features of this Kir7.1 protein with the hydrophibicity, hydropholicity, the propency to generate alpha helix, beta sheet, turn or coiled regions, the propency to be on surface of the protein, and the flexible regions. The boxed areas are the areas which correspond to the regions indicated. The hydrophobicity plot illustrates hydrophobic areas of the protein sequence which are in the lipid bilayer and hydrophilic areas which are outside the lipid bilayer. The antigenicity of the protein fragments is higher in areas exposed to the surface, hydrophilic and flexible. The analysis was performed with Protean (version 3.08a) from DNASTAR.

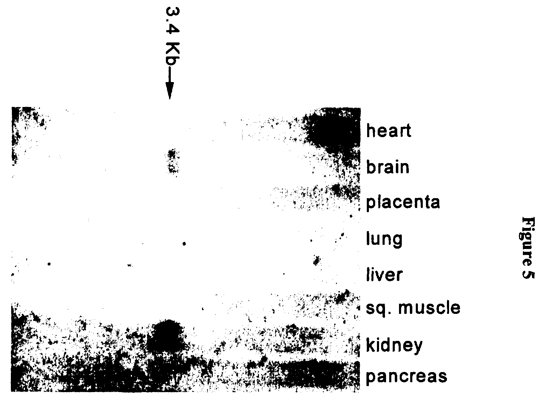

***Figure 5*** shows the expression on a Northern blot of Kir7.1 in human tissues. A Multiple Tissue Northern (MTN) blot (clontech) was hybridized with 32P-labeled BamHI-HindIII 2.0 Kb fragment from the nucleotide sequence Kir7.1 in Express Hyb buffer (Clontech) according to the manufacturer specifications, and washed to a final stringency of 0.1% SDS 55°C. The signal was detected using a Storm (Molecular Dynamics) after a 3 day exposure.

### DESCRIPTION OF THE INVENTION

### Definitions

The following definitions are provided to facilitate understanding of certain terms used frequently herein.

"Receptor Activity" or "Biological Activity of the Receptor" refers to the metabolic or physiologic function of said Kir7.1 including similar activities or improved activities or these activities with decreased undesirable side-effects. Also included are antigenic and immunogenic activities of said Kir7.1

" Kir7.1 polypeptide" refers among others to a polypeptide comprising the amino acid sequence set forth in SEQ ID NO:2 or an allelic variant thereof.

" Kir7.1 gene" or " Kir7.1 polynucleotide" refer to a polynucleotide comprising the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxyribonucleotide, which may be unmodified RNA or DNA or modified RNA or DNA. "Polynucleotides" include, without limitation single- and double-stranded DNA, DNA that is a mixture of single- and double-stranded regions, single-and double-stranded RNA, and RNA that is mixture of single-and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres.

"Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B.C. Johnson, Ed., Academic Press, New York, 1983; Seifter *et al*., "Analysis for protein modifications and nonprotein cofactors", *Meth Enzymol* (1990) 182:626-646 and Rattan *et al*., "Protein Synthesis: Posttranslational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied* *Math* (1988) 48:1073. Methods to determine identity and similarity are codified in computer programs. Preferred computer program methods to determine identity and similarity between two sequences include, but are not limited to, GCS program package (Devereux, J., *et al*., *Nucleic Acids Research* (1984) 12(1):387), BLASTP, BLASTN, FASTA (Atschul, S.F. *et al*., *J Molec Biol* (1990) 215:403). As an illustration, by a polynucleotide having a nucleotide sequence having at least, for example, 95% "identity" to a reference nucleotide sequence of SEQ ID NO : 1 is intended that the nucleotide sequence of the polynucleotide is identical to the reference sequence except that the polynucleotide sequence may include up to five point mutations per each 100 nucleotides of the reference nucleotide sequence of SEQ ID NO : 1. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence is intended that the amino acid sequence of the polypeptide is identical to the reference sequence except that the polypeptide sequence may include up to five amino acid alterations per each 100 amino acids of the reference amino acid of SEQ ID NO : 2. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

### Polynucleotides of the Invention

In accordance with an aspect of this invention, there is provided an isolated nucleic acid (polynucleotide) which encodes for the mature polypeptide having the deduced aminoacid sequence of figure 1b or for the mature polypeptide encoded by the cDNA of the clone deposited with the American Type Culture Collection (ATCC), 12301 Park Lawn Drive, Rockville, Maryland 20852, USA, as ATCC deposit n° 209321 on October 1^{st} 1997.
The deposited material (clone) is a SOLR strain containing the expression bluescript vector from Stratagene that further contains the full length Kir7.1 cDNA, referred to as "Kir7.1" upon deposit. The cDNA insert is within EcoRI-XhoI site(s) in the vector. The nucleotide sequence of the polynucleotides contained in the deposited material, as well as the amino acid sequence of the polypeptide encoded thereby, are controlling in the event of any conflict with any description of sequences herein.
The deposit has been made under the terms of the Budapest Treaty on the international recognition of the deposit of micro-organisms for purposes of patent procedure.

The polynucleotide of this invention was discovered in a cDNA library derived from human brain brain cortex. It is structurally related to the inwardly rectifying potassium (Kir) channel family.

Kir7.1 cDNA contains an open reading frame coding for a protein of 360 amino acids. This gene product contains a N-terminal end and a C-terminal end which are hydrophilic. As predicted by previous analysis on inwardly rectifying potassium channels, the hydrophobycity plot (figure 4) shows the presence of two transmembrane-spanning domains that flank a highly conserved pore (P)-region including the GYG motif (position 121 to 123 in Kir7.1 protein, see figure 3). This region is considered as the signature of K⁺ channels (Heginbotham et al., 1994, Biophys. J. 66, pp 1061-1067). Taken together, these properties show that this protein can be included in the "inwardly rectifying potassium channel subfamily" as confirmed by the dendrogram shown in figure 2, where the Kir7.1 amino acid sequence was compared with other members of the potassium channel family. Computer analyses were performed using the GCG software (Genetics Computer Group). This dendrogram was generated using the PILEUP multiple sequence analysis program with full-length coding sequences for the listed subunits. The gap penalty was 3, and the gap extension penalty was 1. Specific members of subfamilies and their accession numbers are as follows: Kir1.1 (ROMK1, U12541), Kir1.2 (U73191), Kir1.3 (U73193), Kir2.1 (U12507), Kir2.2 (X78461), Kir2.3 (U07364), Kir3.1 (U01071), Kir3.2 (U11859), Kir3.3 (U11860), Kir3.4 (CIR, X83584), Kir4.1 (BIR10, X83585), Kir5.1 (BIR9, X83581), Kir6.1 (D42145), Kir6.2 (D50581), Kv1 (hKv1.1, L02750), Kv2 (mShab, M64228), Kv3 (mKv3.3, X60796), Kv4 (mShal, M64226), BKCa (hSLO, U11717), SKCa1(U69883), SKCa2 (U69882), SKCa3 (U69884), TWIK1 (U33632), and HERG (U36925). Note that the Kir1.2 sequence was defined as the human homologue of the rat sequence Kir4.1 (Shuck et al., 1997, J. Biol. Chem. 272 pp586-593). Alignment was performed with the clustal algorythm of MEGALIGN (version 3.10a ) from DNASTAR.

Unless otherwise indicated, all nucleotide sequences determined by sequencing a DNA molecule herein were determined using an automated DNA sequencer (such as the Model 373 or 377 from Applied Biosystems, Inc.), and all amino acid sequences of polypeptides encoded by DNA molecules determined herein were predicted by translation of a DNA sequence determined as above. Therefore, as is known in the art for any DNA sequence determined by this automated approach, any nucleotide sequence determined herein may contain some errors. Nucleotide sequences determined by automation are typically at least about 90% identical, more typically at least about 95% to at least about 99.9% identical to the actual nucleotide sequence of the sequenced DNA molecule. The actual sequence can be more precisely determined by other approaches including manual DNA sequencing methods well known in the art. As is also known in the art, a single insertion or deletion in a determined nucleotide sequence compared to the actual sequence will cause a frame shift in translation of the nucleotide sequence such that the predicted amino acid sequence encoded by a determined nucleotide sequence will be completely different from the amino acid sequence actually encoded by the sequenced DNA molecule, beginning at the point of such an insertion or deletion.

As one of ordinary skill would appreciate, however, due to the possibilities of sequencing errors, as well as the variability of cleavage sites for leaders in different known proteins, the full-length Kir7.1 polypeptide comprises about 360 amino acids, but may be anywhere in the range of about 330 to about 400 amino acids.

As indicated, nucleic acid molecules of the present invention may be in the form of RNA, such as mRNA, or in the form of DNA, including, for instance, cDNA and genomic DNA obtained by cloning or produced synthetically. The DNA may be double-stranded or single-stranded. Single-stranded DNA or RNA may be the coding strand, also known as the sense strand, or it may be the non-coding strand, also referred to as the anti-sense strand.

By "isolated" nucleic acid molecule(s) is intended a nucleic acid molecule, DNA or RNA, which has been removed from its native environment. For example, recombinant DNA molecules contained in a vector are considered isolated for the purposes of the present invention. Further examples of isolated DNA molecules include recombinant DNA molecules maintained in heterologous host cells or purified (partially or substantially) DNA molecules in solution. Isolated RNA molecules include *in vivo* or *in vitro* RNA transcripts of the DNA molecules of the present invention. Isolated nucleic acid molecules according to the present invention further include such molecules produced synthetically. Isolated nucleic acid molecules of the present invention include DNA molecules comprising the open reading frame (ORF) shown in figure 1a and DNA molecules which comprise a sequence substantially different from those described above but which, due to the degeneracy of the genetic code, still encode the Kir7.1 channel. Of course, the genetic code is well known in the art. Thus, it would be routine for one skilled in the art to generate such degenerate variants.

In another aspect, the invention provides isolated nucleic acid molecules encoding the Kir7.1 polypeptide having an amino acid sequence encoded by the cDNA set forth in figure 1a. In further embodiments, this nucleic acid molecule will encode the mature polypeptide or the full-length polypeptide lacking the N-terminal methionine. The invention further provides an isolated nucleic acid molecule having the nucleotide sequence shown in Figure 1a or the nucleotide sequence of the Kir7.1 channel cDNA contained in the above-described deposited clone, or a nucleic acid molecule having a sequence complementary to one of the above sequences. Such isolated molecules, particularly DNA molecules, are useful as probes for gene mapping, by in situ hybridization with chromosomes, and for detecting expression of the Kir7.1 channel gene in human tissue, for instance, by Northern blot analysis.

The present invention is further directed to fragments of the isolated nucleic acid molecules described herein. By a fragment of an isolated nucleic acid molecule having the nucleotide sequence of the deposited cDNA or the nucleotide sequence shown in Figure 1a is intended fragments at least about 15 nucleotides, and more preferably at least about 20 nucleotides, still more preferably at least about 30 nucleotides, and even more preferably, at least about 40 nucleotides in length which are useful as diagnostic probes and primers as discussed herein. Of course, larger fragments 50-1500 nucleotides in length are also useful according to the present invention, as are fragments corresponding to most, if not all, of the nucleotide sequence of the deposited cDNA or as shown in Figure 1a. By a fragment at least 20 nucleotides in length, for example, is intended fragments which include 20 or more contiguous bases from the nucleotide sequence of the deposited cDNA or the nucleotide sequence as shown in Figure 1a.

Preferred nucleic acid fragments of the present invention include nucleic acid molecules encoding: a polypeptide comprising the Kir7.1 channel extracellular domain, a polypeptide comprising the Kir7.1 channel transmembrane domains, a polypeptide comprising the Kir7.1 channel intracellular domain and a polypeptide comprising the Kir7.1 extracellular and intracellular domains with all or part of the transmembrane domain deleted. As above with the leader sequence, the amino acid residues constituting the Kir7.1 channel extracellular, transmembrane and intracellular domains have been predicted by computer analysis. Thus, as one of ordinary skill would appreciate, the amino acid residues constituting these domains may vary slightly (e.g., by about 1 to about 15 amino acid residues) depending on the criteria used to define each domain.

Preferred nucleic acid fragments of the present invention also include nucleic acid molecules encoding epitope-bearing portions of the Kir7.1 channel protein.

In another aspect, the invention provides an isolated nucleic acid molecule comprising a polynucleotide which hybridizes under stringent hybridization conditions to a portion of the polynucleotide in a nucleic acid molecule of the invention described above, for instance, the full-length cDNA set forth in Figure 1a or the cDNA clone contained in ATCC Deposit n° 209321. By "stringent hybridization conditions" is intended overnight incubation at 42°C in a solution comprising: 50% formamide, 5x SSC (150 mM NaCl, 15mM trisodium citrate), 50 mM sodium phosphate (pH 7.6), 5x Denhardt's solution, 10% dextran sulfate, and 20 g/ml denatured, sheared salmon sperm DNA, followed by washing the filters in 0.1x SSC at about 65°C.

By a polynucleotide which hybridizes to a "portion" of a polynucleotide is intended a polynucleotide (either DNA or RNA) hybridizing to at least about 15 nucleotides (nt), and more preferably at least about 20 nt, still more preferably at least about 30 nt, and even more preferably about 30-70 nt of the reference polynucleotide. These are useful as diagnostic probes and primers as discussed above and in more detail below.

By a portion of a polynucleotide of "at least 20 nt in length," for example, is intended 20 or more contiguous nucleotides from the nucleotide sequence of the reference polynucleotide (e.g., the deposited cDNA or the nucleotide sequence as shown in Figure 1a).

Of course, a polynucleotide which hybridizes only to a poly A sequence (such as the 3', terminal poly(A) tract of the Kir7.1 channel cDNA shown in Figure 1a), or to a complementary stretch of T (or U) resides, would not be included in a polynucleotide of the invention used to hybridize to a portion of a nucleic acid of the invention, since such a polynucleotide would hybridize to any nucleic acid molecule containing a poly (A) stretch or the complement thereof (e.g., practically any double-stranded cDNA clone).

As indicated, nucleic acid molecules of the present invention which encode a Kir7.1 polypeptide may include, but are not limited to those encoding the amino acid sequence of the mature polypeptide, by itself; the coding sequence for the mature polypeptide and additional sequences, such as a pre-, or pro- or prepro- protein sequence; the coding sequence of the mature polypeptide, with or without the aforementioned additional coding sequences, together with additional, non-coding sequences, including for example, but not limited to introns and non-coding 5' and 3' sequences, such as the transcribed, non-translated sequences that play a role in transcription, mRNA processing, including splicing and polyadenylation signals, for example ribosome binding and stability of mRNA; an additional coding sequence which codes for additional amino acids, such as those which provide additional functionalities. Thus, the sequence encoding the polypeptide may be fused to a marker sequence, such as a sequence encoding a peptide which facilitates purification of the fused polypeptide. In certain preferred embodiments of this aspect of the invention, the marker amino acid sequence is a hexa-histidine peptide, such as the tag provided in a pQE vector (Qiagen, Inc.), among others, many of which are commercially available. As described in Gentz *et al., Proc. Natl. Acad. Sci. USA 86*: 821-824 (1989), for instance, hexa-histidine provides for convenient purification of the fusion protein. The "HA" tag is another peptide useful for purification which corresponds to an epitope derived from the influenza hemagglutinin protein, which has been described by Wilson *et al., Cell 37*: 767 (1984). As discussed below, other such fusion proteins include the Kir7.1 channel fused to the maltose binding protein sequence, or to Fc at the N- or C-terminus.

The present invention further relates to variants of the nucleic acid molecules of the present invention, which encode portions, analogs or derivatives of the Kir7.1 channel. Variants may occur naturally, such as a natural allelic variant. By an "allelic variant" is intended one of several alternate forms of a gene occupying a given locus on a chromosome of an organism. Genes II, Lewin, B., ed., John Wiley & Sons, New York (1985). Non-naturally occurring variants may be produced using art-known mutagenesis techniques.

Such variants include those produced by nucleotide substitutions, deletions or additions, which may involve one or more nucleotides. The variants may be altered in coding regions, non-coding regions, or both. Alterations in the coding regions may produce conservative or non-conservative amino acid substitutions, deletions or additions. Especially preferred among these are silent substitutions, additions and deletions, which do not alter the properties and activities of the Kir7.1 channel or portions thereof. Also especially preferred in this regard are conservative substitutions.

Further embodiments of the invention include isolated nucleic acid molecules comprising a polynucleotide having a nucleotide sequence at least 80%, preferably 90% , and more preferably at least 95%, 96%, 97%, 98% or 99% identical to (a) a nucleotide sequence encoding the polypeptide having the complete amino acid sequence in figure 1b; (b) a nucleotide sequence encoding the polypeptide having the complete amino acid sequence in Figure 1b except for the N-terminal methionine (amino acid residues 2 to 360 in Figure 1b); (c) a nucleotide sequence encoding the Kir7.1 polypeptide having the amino acid sequence encoded by the cDNA clone contained in ATCC Deposit n° 209321 (d) a nucleotide sequence encoding the Kir7.1 channel extracellular domain; (e) a nucleotide sequence encoding the Kir7.1 channel transmembrane domain; (f) a nucleotide sequence encoding the Kir7.1 channel intracellular domain; (g) a nucleotide sequence encoding the Kir7.1 channel extracellular and intracellular domains with all or part of the transmembrane domain deleted; and (h) a nucleotide sequence complementary to any of the nucleotide sequences in (a), (b), (c), (d), (e), (f), (g), or (h).

As a practical matter, whether any particular nucleic acid molecule is at least 90%, 95%, 96%, 97%, 98% or 99% identical to, for instance, the nucleotide sequence shown in Figure 1a or to the nucleotides sequence of the deposited cDNA clone can be determined conventionally using known computer programs such as the Bestfit program (Wisconsin Sequence Analysis Package, Version 9 for Unix, Genetics Computer Group, University Research Park, 575 Science Drive, Madison, WI 53711). Bestfit uses the local homology algorithm of Smith and Waterman, *Advances in Applied Mathematics 2*: 482-489 (1981), to find the best segment of homology between two sequences. When using Bestfit or any other sequence alignment program to determine whether a particular sequence is, for instance, 95% identical to a reference sequence according to the present invention, the parameters are set, of course, such that the percentage of identity is calculated over the full length of the reference nucleotide sequence and that gaps in homology of up to 5% of the total number of nucleotides in the reference sequence are allowed.

The present application is directed to nucleic acid molecules at least 80%, 90%, 95%, 96%, 97%, 98% or 99% identical to the nucleic acid sequence shown in Figure 1a or to the nucleic acid sequence of the deposited cDNA, irrespective of whether they encode a polypeptide having Kir7.1 channel activity. This is because even where a particular nucleic acid molecule does not encode a polypeptide having Kir7.1 channel activity, one of skill in the art would still know how to use the nucleic acid molecule, for instance, as a hybridization probe or a polymerase chain reaction (PCR) primer. Uses of the nucleic acid molecules of the present invention that do not encode a polypeptide having Kir7.1 channel activity include, inter alia, (1) isolating the Kir7.1 channel gene or allelic variants thereof in a cDNA library; (2) in situ hybridization (e.g., "FISH") to metaphase chromosomal spreads to provide precise chromosomal location of the Kir7.1 channel gene, as described in Verma *et al., Human Chromosomes: A Manual of Basic Techniques*, Pergamon Press, New York (1988); and (3) Northern Blot analysis for detecting Kir7.1 channel mRNA expression in specific tissues.

Preferred, however, are nucleic acid molecules having sequences at least 90%, 95%, 96%, 97%, 98% or 99% identical to the nucleic acid sequence shown in Figure 1a or to the nucleic acid sequence of the deposited cDNA which do, in fact, encode a polypeptide having Kir7.1 channel activity.

Of course, due to the degeneracy of the genetic code, one of ordinary skill in the art will immediately recognize that a large number of the nucleic acid molecules having a sequence at least 80%, 90%, 95%, 96%, 97%, 98%, or 99% identical to the nucleic acid sequence of the deposited cDNA or the nucleic acid sequence shown in Figure 1a will encode a polypeptide "having Kir7.1 channel activity." It will be further recognized in the art that, for such nucleic acid molecules that are not degenerate variants, a reasonable number will also encode a polypeptide having Kir7.1 protein activity. This is because the skilled artisan is fully aware of amino acid substitutions that are either less likely or not likely to significantly effect protein function (e.g., replacing one aliphatic amino acid with a second aliphatic amino acid).

For example, guidance concerning how to make phenotypically silent amino acid substitutions is provided in Bowie, J. U. *et al*., *"Deciphering the Message in Protein Sequences: Tolerance to Amino Acid Substitutions*," Science 247: 1306-1310 (1990), wherein the authors indicate that proteins are surprisingly tolerant of amino acid substitutions.

### Polypeptides of the Invention

The invention further provides an isolated Kir7.1 polypeptide having the amino acid sequence encoded by the deposited cDNA ATCC 209321, or the amino acid sequence SEQ ID NO:2 in Figure 1b, or a peptide or polypeptide comprising a portion of the above polypeptides.

It will be recognized in the art that some amino acid sequences of the Kir7.1 channel can be varied without significant effect on the structure or function of the protein. If such differences in sequence are contemplated, it should be remembered that there will be critical areas on the protein which determine activity.

Thus, the invention further includes variations of the Kir7.1 channel which show substantial Kir7.1 channel activity or which include regions of Kir7.1 protein. Such mutants include deletions, insertions, inversions, repeats, and type substitutions. As indicated above, guidance concerning which amino acid changes are likely to be phenotypically silent can be found in Bowie J.U. *et al.,* "Deciphering the Message in Protein Sequences: Tolerance to Amino Acid Substitutions," *Science 247*: 1306-1310 (1990).

Thus, the fragment, derivative or analog of the polypeptide SEQ ID NO:2 (Figure 1b), or that encoded by the deposited cDNA, may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as an IgG Fc fusion region peptide or leader or secretory sequence or a sequence which is employed for purification of the mature polypeptide or a proprotein sequence. Such fragments, derivatives and analogs are deemed to be within the scope of those skilled in the art from the teachings herein.

Of particular interest are substitutions of charged amino acids with another charged amino acid and with neutral or negatively charged amino acids. The latter results in proteins with reduced positive charge to improve the characteristics of the Kir7.1 protein. The prevention of aggregation is highly desirable. Aggregation of proteins not only results in a loss of activity but can also be problematic when preparing pharmaceutical formulations, because they can be immunogenic (Pinckard *et al., Clin. Exp. Immunol. 2*: 331-340 (1967); Robbins *et al., Diabetes 36*: 838-845 (1987); Cleland *et al*. *Crit. Rev. Therapeutic Drug Carrier Systems 10*: 307-377 (1993)).

The replacement of amino acids can also change the selectivity of binding to cell surface receptors. Ostade *et al*. (*Nature 361*: 266-268 (1993)) describes certain mutations resulting in selective binding of TNF-α to only one of the two known types of TNF receptors. Thus, the Kir7.1 channel of the present invention may include one or more amino acid substitutions, deletions or additions, either from natural mutations or human manipulation.

As indicated, changes are preferably of a minor nature, such as conservative amino acid substitutions that do not significantly affect the folding or activity of the protein (see Table 1).

**TABLE 1**

| **Conservative Amino Acid Substitutions.** | |
|---|---|
| Aromatic | Phenylalanine |
| | Tryptophan |
| | Tyrosine |
| Hydrophobic | Leucine |
| | Isoleucine |
| | Valine |
| Polar | Glutamine |
| | Asparagine |
| Basic | Arginine |
| | Lysine |
| | Histidine |
| Acidic | Aspartic Acid |
| | Glutamic Acid |
| Small | Alanine |
| | Serine |
| | Threonine |
| | Methionine |
| | Glycine |

Amino acids in the Kir7.1 protein of the present invention that are essential for function can be identified by methods known in the art, such as site-directed mutagenesis or alanine-scanning mutagenesis (Cunningham and Wells, *Science* *244*: 1081-1085 (1989)). The latter procedure introduces single alanine mutations at every residue in the molecule. The resulting mutant molecules are then tested for biological activity such as receptor binding or *in vitro*, or *in vitro* proliferative activity. Sites that are critical for ligand-receptor binding can also be determined by structural analysis such as crystallization, nuclear magnetic resonance or photoaffinity labeling (Smith *et al., J. Mol. Biol. 224*: 899-904 (1992) and de Vos *et al. Science 255*: 306-312 (1992)).

The polypeptides of the present invention are preferably provided in an isolated form. By "isolated poylypeptide" is intended a polypeptide removed from its native environment. Thus, a polypeptide produced or contained in a recombinant host cell is considered "isolated" for the purposes of the present invention. Also intended as "isolated" is a polypeptide that has been purified, partially or substantially, from a recombinant host. For example, a recombinantly produced version of the Kir7.1 channel can be substantially purified by the one-step method described in Smith and Johnson, *Gene 67*: 31-40 (1988).

The polypeptides of the present invention also include the complete polypeptide encoded by the deposited cDNA; the mature polypeptide encoded by the deposited the cDNA; amino acid residues 1 to 360 of Figure 1b; the extracellular domain; the transmembrane domains; and the intracellular domains, as well as polypeptides which are at least 80% identical, more preferably at least 90% or 95% identical, still more preferably at least 96%, 97%, 98% or 99% identical to the polypeptides described above, and also include portions of such polypeptides with at least 30 amino acids and more preferably at least 50 amino acids.

### Vectors and Host Cells

The present invention also relates to vectors which comprise isolated polynucleotide or polynucleotides of the present invention, host cells which are genetically engineered with the recombinant vectors, and the production of Kir7.1 polypeptides or fragments thereof by recombinant techniques.

Thus, the invention also relates to a DNA or RNA molecule comprising an expression system, wherein said expression system is capable of producing a Kir7.1 polypeptide comprising an amino acid sequence, which has at least 80% identity with the polypeptide of SEQ ID NO:2 when said expression system is present in a compatible host cell.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged *in vitro* using an appropriate packaging cell line and then transduced into host cells.

The DNA insert should be operatively linked to an appropriate promoter, such as the phage lambda PL promoter, the *E. coli lac*, *trp* and *tac* promoters, the SV40 early and late promoters and promoters of retroviral LTRs, to name a few. Other suitable promoters will be known to the skilled artisan. The expression constructs will further contain sites for transcription initiation, termination and, in the transcribed region, a ribosome binding site for translation. The coding portion of the mature transcripts expressed by the constructs will preferably include a translation initiating at the beginning and a termination codon (UAA, UGA or UAG) appropriately positioned at the end of the polypeptide to be translated.

As indicated, the expression vectors will preferably include at least one selectable marker. Such markers include dihydrofolate reductase , zeocin, hygromycin, or neomycin resistance for eukaryotic cell culture and tetracycline or ampicillin resistance genes for culturing in *E. coli* and other bacteria. Representative examples of appropriate heterologous hosts include, but are not limited to, bacterial cells, such as *E. coli*, *Streptomyces* and *Salmonella typhimurium* cells; fungal cells, such as yeast cells; insect cells such as *Drosophila* S2 and *Spodoptera* Sf9 cells; animal cells such as CHO, COS and Bowes melanoma cells; and plant cells. Appropriate culture mediums and conditions for the above-described host cells are known in the art.

Among vectors preferred for use in bacteria include pQE70, pQE60 and pQE-9, available from Qiagen; pBS vectors, Phagescript vectors, Bluescript vectors, pNH8A, pNH16a, pNH18A, pNH46A, available from Stratagene; and ptrc99a, pKK223-3, pKK233-3, pDR540, pRIT5 available from Pharmacia. Among preferred eukaryotic vectors are pWLNEO, pSV2CAT, pOG44, pXT1 and pSG available from Stratagene; and pSVK3, pBPV, pMSG and pSVL available from Pharmacia. Other suitable vectors will be readily apparent to the skilled artisan.

Introduction of the expression system into the host cell can be effected by known transforming or transfecting. Thus, the introduction of the expression system methods may be effected by calcium phosphate transfection, DEAE-dextran mediated transfection, cationic lipid-mediated transfection, electroporation, transduction, infection or other methods. Such methods are described in many standard laboratory manuals, such as Davis *et al., Basic Methods In Molecular Biology* (1986).

The polypeptide may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification, or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP-A-O 464 533 (Canadian counterpart 2045869) discloses fusion proteins comprising various portions of constant region of immunoglobulin molecules together with another human protein or part thereof. In many cases, the Fc part in a fusion protein is thoroughly advantageous for use in therapy and diagnosis and thus results, for example, in improved pharmacokinetic properties (EP-A 0232 262). On the other hand, for some uses it would be desirable to be able to delete the Fc part after the fusion protein has been expressed, detected and purified in the advantageous manner described. This is the case when Fc portion proves to be a hindrance to use in therapy and diagnosis, for example when the fusion protein is to be used as antigen for immunizations. In drug discovery, for example, human proteins, such as, hIL5- has been fused with Fc portions for the purpose of high-throughput screening assays to identify antagonists of hIL-5. See, D. Bennett *et al., Journal of* *Molecular Recognition, Vol. 8*:52-58 (1995) and K. Johanson *et al., The Journal of Biological Chemistry, Vol. 270*, No. 16:9459-9471 (1995).

The Kir7.1 receptor can be recovered and purified from recombinant cell cultures by well-known methods, including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification. Polypeptides of the present invention include naturally purified products, products of chemical synthetic procedures, and products produced by recombinant techniques from a prokaryotic or eukaryotic host, including, for example, bacterial, yeast, higher plant, insect and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes.

### Diagnostic Assays

This invention also relates to the use of Kir7.1 polynucleotides for use as diagnostic reagents. Detection of a mutated form of Kir7.1 gene associated with a dysfunction will provide a diagnostic tool that can add to or define a diagnosis of a disease or susceptibility to a disease which results from under-expression, over-expression or altered expression of Kir7.1.Individuals carrying mutations in the Kir7.1 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled Kir7.1 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising Kir7.1 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The diagnostic assays offer a process for diagnosing or determining a susceptibility to the treatment of diseases through detection of mutation in the Kir7.1 gene by the methods described. Said diseases are, in particular, chosen among the following : cerebral and cardiac and renal ischemias, brain and cardiac diseases, auto-immune diseases and inflammation, pain, cell proliferation disorders such as cancer, decrease or increase of endogenous neurotransmitters and hormones releases, disorder related to abnormal expression of the Kir7.1 protein, more particularly in brain and kidneys.

In addition, the same diseases can be diagnosed by methods comprising determining from a sample derived from a subject an abnormally decreased or increased level of Kir7.1 polypeptide or Kir7.1 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an Kir7.1, in a sample derived from a host are well-known to those of skill in the art. Such assay methods include radioimmunoassays, competitive-binding assays, Western Blot analysis and ELISA assays.

### Detection of Kir7.1 Gene Expression

The expression level of the *Kir7.1* gene can be readily assayed by one of ordinary skill in the art. By "assaying the expression level of the gene encoding the Kir7.1 polypeptide" is intended qualitatively or quantitatively measuring or estimating the level of the Kir7.1 polypeptide or the level of the mRNA encoding the Kir7.1 polypeptide in a biological sample (e.g., by determining or estimating absolute protein level or mRNA level).

By "biological sample" is intended any biological sample obtained from an individual, cell line, tissue culture, or other source which contains Kir7.1 polypeptide or Kir7.1 mRNA. Such tissues include cerebellum, brain, midbrain, spinal cord, nerve endings, retina, breast, pituitary, heart, placenta, lung, skeletal muscle, kidney, and pancreas. Biological samples include mammalian tissues which contain Kir7.1 polypeptide. Preferred mammals include monkeys, apes, cats, dogs, cows, pigs, horses, rabbits, and humans. Particularly preferred are humans.

Total cellular RNA can be isolated from a biological sample using the single-step guanidinium-thiocyanate-phenol-chloroform method described in Chomczynski and Sacchi (*Anal*. *Biochem. 162*:156-159 (1987)). Levels of mRNA encoding the Kir7.1 receptor are then assayed using any appropriate method. These include Northern blot analysis (Harada *et al., Cell 63*:303-312 (1990)), S1 nuclease mapping (Harada *et al., Cell 63*:303-312 (1990)), the polymerase chain reaction (PCR), reverse transcription in combination with the polymerase chain reaction (RT-PCR) (Fujita *et al., Cell 49*:35-36 (1990)), and reverse transcription in combination with the ligase chain reaction (RT-LCR).

As discussed here-above, assaying Kir7.1 polypeptide levels in a biological sample can occur using antibody-based techniques. For example, Kir7.1 polypeptide expression in tissues can be studied with classical immunohistological methods (Jalkanen, M. *et al., J. Cell. Biol. 101*:976-985 (1985); Jalkanen, M. *et al., J. Cell. Biol. 105*: 3087-3096 (1987)). Other antibody-based methods useful for detecting Kir7.1 polypeptide gene expression include immunoassays, such as the enzyme linked immunosorbent assay (ELISA) and the radioimmunoassay (RIA). Suitable labels are known in the art and include enzyme labels, such as glucose oxidase, and radioisotopes, such as iodine (¹²⁵I, ¹²¹I), carbon (¹⁴C), sulfur (³⁵S), tritium (³H), indium (¹¹²In), and technetium (^{99m}Tc), biotin and fluorescent labels, such as fluorescein and rhodamine.

### Chromosome Assays

The nucleic acid molecules of the present invention are also valuable for chromosome identification. The sequence is specifically targeted to and can hybridize with a particular location on an individual human chromosome. The mapping of DNAs to chromosomes according to the present invention is an important first step in correlating those sequences with genes associated with disease.

In certain preferred embodiments in this regard, the cDNA herein disclosed is used to clone genomic DNA of a Kir7.1 polypeptide gene. This can be accomplished using a variety of well known techniques and libraries, which generally are available commercially. The genomic DNA then is used for *in situ* chromosome mapping using well known techniques for this purpose.

In addition, in some cases, sequences can be mapped to chromosomes by preparing PCR primers (preferably 15-25 bp) from the cDNA. Computer analysis of the 3' untranslated region of the gene is used to rapidly select primers that do not span more than one exon in the genomic DNA, thus complicating the amplification process. These primers are then used for PCR screening of somatic cell hybrids containing individual human chromosomes.

Fluorescence in situ hybridization ("FISH") of a cDNA clone to a metaphase chromosomal spread can be used to provide a precise chromosomal location in one step. This technique can be used with probes from the cDNA as short as 50 or 60 bp. For a review of this technique, see Verma *et al., Human Chromosomes: A Manual Of Basic Techniques*, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance In Man, available on-line through Johns Hopkins University, Welch Medical Library. The relationship between genes and diseases that have been mapped to the same chromosomal region are then identified through linkage analysis (coinheritance of physically adjacent genes). Next, it is necessary to determine the differences in the cDNA or genomic sequence between affected and unaffected individuals. If a mutation is observed in some or all of the affected individuals but not in any normal individuals, then the mutation is likely to be the causative agent of the disease.

### Antibodies

The Kir7.1 polypeptides of the present invention can be used to generate antibodies. Such antibodies can be used to investigate the expression, regulation, and ligand binding properties of the Kir7.1 channel.

In another aspect, the invention provides a peptide or polypeptide comprising an epitope-bearing portion of a polypeptide of the invention. The epitope of this polypeptide portion is an immunogenic or antigenic epitope of a polypeptide described herein. An "immunogenic epitope" is defined as a part of a protein that elicits an antibody response when the whole protein is the immunogen. On the other hand, a region of a protein molecule to which an antibody can bind is defined as an "antigenic epitope". The number of immunogenic epitopes of a protein generally is less than the number of antigenic epitopes. See, for instance, Geysen *et al., Proc. Natl. Acad. Sci. USA 81*: 3998- 4002 (1983).

As to the selection of peptides or polypeptides bearing an antigenic epitope (i.e., that contain a region of a protein molecule to which an antibody can bind), it is well known in that art that relatively short synthetic peptides that mimic part of a protein sequence are routinely capable of eliciting an antiserum that reacts with the partially mimicked protein (See, for instance, Sutcliffe J. G., Shinnick T. M., Green N. and Learner R. A., Antibodies that react with predetermined sites on protein, *Science 219*: 660-666 (1983)). Peptides capable of eliciting protein-reactive sera are frequently represented in the primary sequence of a protein, can be characterized by a set of simple chemical rules, and are confined neither to immunodominant regions of intact proteins (i.e., immunogenic epitopes) nor to the amino or carboxyl terminals. Antigenic epitope-bearing peptides and polypeptides of the invention are therefore useful to raise antibodies, including monoclonal antibodies, that bind specifically to a polypeptide of the invention (See, for instance, Wilson *et al., Cell 37*:767-778 (1984)).

Antigenic epitope-bearing peptides and polypeptides of the invention preferably contain a sequence of at least seven, more preferably at least nine and most preferably between at least about 15 to about 30 amino acids contained within the amino acid sequence of a polypeptide of the invention. Non-limiting examples of antigenic polypeptides or peptides that can be used to generate Kir7.1 channel-specific antibodies include: a polypeptide comprising amino acid residues from about 13 to about 28 in Figure 1b; a polypeptide comprising amino acid residues from about 30 to about 38 in Figure 1b; a polypeptide comprising amino acid residues from about 81 to about 96 in Figure 1b; a polypeptide comprising amino acid residues from about 127 to about 135 in Figure 1b; a polypeptide comprising amino acid residues from about 160 to about 169 in Figure 1b; a polypeptide comprising amino acid residues from about 180 to about 188 in Figure 1b; a polypeptide comprising amino acid residues from about 193 to about 199 in Figure 1b; a polypeptide comprising amino acid residues from about 209 to about 219 in Figure 1b; a polypeptide comprising amino acid residues from about 224 to about 236 in Figure 1b; a polypeptide comprising amino acid residues from about 259 to about 266 in Figure 1b; a polypeptide comprising amino acid residues from about 275 to about 292 in Figure 1b; a polypeptide comprising amino acid residues from about 303 to about 324 in Figure 1b; a polypeptide comprising amino acid residues from about 331 to about 340 in Figure 1b and a polypeptide comprising amino acid residues from about 343 to about 353 in Figure 1b. As indicated above, the inventors have determined that the above polypeptide fragments are antigenic regions of the Kir7.1 channel protein. The epitope-bearing peptides and polypeptides of the invention may be produced by any conventional means (See, for instance, Houghten R. A., General method for the rapid solid-phase synthesis of large numbers of peptides: Specificity of antigen-antibody interaction at the level of individual amino acids, *Proc. Natl. Acad. Sci. USA 82*: 5131-5135 (1985). This "Simultaneous Multiple Peptide Synthesis (SMPS)" process is further described in U.S. Patent No. 4,631,211 to Houghten et al. (1986)).

As one of skill in the art will appreciate, Kir7.1 polypeptides of the present invention and the epitope-bearing fragments thereof described above can be combined with parts of the constant domain of immunoglobulins (IgG), resulting in chimeric polypeptides. These fusion proteins facilitate purification and show an increased half-life *in vivo*. This has been shown, e.g., for chimeric proteins consisting of the first two domains of the human CD4-polypeptide and various domains of the constant regions of the heavy or light chains of mammalian immunoglobulins (EPA 394,827; Traunecker *et al., Nature 331*: 84- 86 (1988)). Fusion proteins that have a disulfide-linked dimeric structure due to the IgG part can also be more efficient in binding and neutralizing other molecules than the monomeric Kir7.1 protein or protein fragment alone (Fountoulakis *et al., J. Biochem. 270*: 3958-3964 (1995)).

The above-mentioned antibodies may be employed to isolate or to identify clones expressing the polypeptide or to purify the polypeptides by affinity chromatography.

Antibodies against Kir7.1 polypeptides may also be employed to treat, among others, patients with cerebral and cardiac and renal ischemias, brain and cardiac diseases, auto-immune diseases and inflammation, pain, cell proliferation disorders such as cancer, to mimic or antagonize effect of endogenous neurotransmitters and hormones, to decrease or increase endogenous neurotransmitters and hormones releases, or to diagnose or treat any disorder related to abnormal expression of the Kir7.1 polypeptides, more particularly in brain and kidneys.

### Screening Assays

The Kir7.1 polypeptide of the present invention may be employed in a screening process for compounds which bind the receptor and which activate (agonists) or inhibit activation of (antagonists) the receptor polypeptide of the present invention. Thus, polypeptides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics. See Coligan *et al., Current Protocols in Immunology* 1(2):Chapter 5 (1991). Kir7.1 polypeptides may be responsible for many biological functions, including many pathologies. Accordingly, it is desirous to find compounds and drugs which stimulate Kir7.1 on the one hand and which can inhibit the function of Kir7.1 on the other hand. In general, agonists are employed for therapeutic and prophylactic purposes for such conditions as, among others, nervous system disorders, to treat cerebral and cardiac and renal ischemias, other brain and cardiac diseases, auto-immune diseases , inflammation, pain, cell proliferation disorders such as cancer; to mimic or antagonize the effects of endogenous neurotransmitters and hormones; to diagnose or treat any disorder related to abnormal expression of this channel protein, more particularly in brain and kidney.

Antagonists may be employed for a variety of therapeutic and prophylactic purposes for such conditions as, among others, nervous system disorders, cerebral and cardiac and renal ischemias, other brain and cardiac diseases, auto-immune diseases , inflammation, pain, cell proliferation disorders such as cancer; to mimic or antagonize the effects of endogenous neurotransmitters and hormones; to diagnose or treat any disorder related to abnormal expression of this channel protein, more particularly in brain and kidney.

In general, such screening procedures involve producing appropriate cells which express the receptor polypeptide of the present invention on the surface thereof. Such cells include cells from mammals, yeast, *Drosophila* or *E. coli*. Cells expressing the receptor (or cell membrane containing the expressed receptor) are then contacted with a test compound to observe binding, or stimulation or inhibition of a functional response.

The assays may simply test binding of a candidate compound wherein adherence to the cells bearing the receptor is detected by means of a label directly or indirectly associated with the candidate compound or in an assay involving competition with a labeled competitor. Further, these assays may test whether the candidate compound results in a signal generated by activation of the receptor, using detection systems appropriate to the cells bearing the receptor at their surfaces. Inhibitors of activation are generally assayed in the presence of a known agonist and the effect on activation by the agonist by the presence of the candidate compound is observed. Standard methods for conducting such screening assays are well understood in the art.

The recording of channel activity may be carried out either by membrane voltage analysis, directly (patch clamp for example) or indirectly (fluorescent probes for example), or by sodium entry measurement (radioactive sodium influx, fluorescent probes or reporter genes for example).

Examples of potential Kir7.1 antagonists include antibodies or, in some cases, oligonucleotides or proteins which are closely related to the ligand of the Kir7.1, e.g., a fragment of the ligand, or small molecules which bind to the receptor but do not elicit a response, so that the activity of the receptor is prevented.

The agonists and antagonists which may be identified by the above screening methods constitute a further aspect of the present invention.

### Prophylactic and Therapeutic Methods

This invention provides methods of treating an abnormal conditions related to both an excess of and insufficient amounts of Kir7.1 activity.

If the activity of Kir7.1 is in excess, several approaches are available. One approach comprises administering to a subject an inhibitor compound (antagonist) as hereinabove described along with a pharmaceutically acceptable carrier in an amount effective to inhibit activation by blocking binding of ligands to the Kir7.1, or by inhibiting a second signal, and thereby alleviating the abnormal condition. In another approach, soluble forms of Kir7.1 polypeptides still capable of binding the ligand in competition with endogenous Kir7.1 may be administered. Typical embodiments of such competitors comprise fragments of the Kir7.1 polypeptide.

In still another approach, expression of the gene encoding endogenous Kir7.1 can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo*.

For treating abnormal conditions related to an under-expression of Kir7.1 and its activity, several approaches are also available. One approach comprises administering to a subject a therapeutically effective amount of a compound which activates Kir7.1, i.e., an agonist as described above, in combination with a pharmaceutically acceptable carrier, to thereby alleviate the abnormal condition. Alternatively, gene therapy may be employed to effect the endogenous production of Kir7.1 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo*. For overview of gene therapy, see Chapter 20, *Gene Therapy and other Molecular Genetic-based Therapeutic Approaches*, (and references cited therein) in Human Molecular Genetics, T Strachan and A P Read, BIOS Scientific Publishers Ltd (1996).

### Examples

The examples below are carried out using standard techniques, which are well known and routine to those of skill in the art, except where otherwise described in detail. The examples illustrate, but do not limit the invention.

### Example 1

### Cloning the Human Kir7.1 potassium Channel

The sequence of the Kir7.1 potassium channel was first identified by searching a database containing approximately 1 million human ESTs, which was generated using high throughput automated DNA sequence analysis of randomly selected human cDNA clones (Adams M. D. *et al., Nature 377*: 3-174 (1995); Adams M.D. *et al., Nature 355*: 632-634 (1992); and Adams M.D. *et al., Science 252*: 1651-1656 (1991)). Sequence homology comparisons of each EST were performed against the GenBank database using the blastn and tblastn algorithms (Altschul S.F. *et al., J. Mol. Biol. 215*: 403-410 (1990)). A specific homology search using the known human Kir6.2 amino acid sequence against this human EST database revealed one EST, from a brain cortex cDNA library, with approximatively 40% similarity to Kir6.2. The sequence comparison of said EST suggested that it contained the complete open reading frame of a new protein. Sequence of the gene was confirmed by double strand DNA sequencing using the TaqFs (Perkin Elmer) and the gene was shown to be completely new by a blast search against Genbank release 101.The entire Kir7.1 coding region containing the EcoRI-XhoI fragments was inserted into the expression vector bluescript (Stratagene) according to methods well known by one skilled in the art.

### Example 2

### Cloning and Expression of Kir7.1 in Mammalian Cells

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as PSVL and PMSG (Pharmacia, Uppsala, Sweden), pRSVcat (ATCC 37152), pSV2dhfr (ATCC 37146) and pBC12MI (ATCC 67109) and pcDNA3 (Invitrogen). Mammalian host cells that could be used include, human HEK 293, H9 and Jurkat cells, mouse NIH3T3 and C127 cells, Cos 1, Cos 7 and CV 1, quail QC1-3 cells, mouse L cells and Chinese hamster ovary (CHO) cells ; all of these cells being commercially available.

Alternatively, the gene can be expressed in stable cell lines that contain the gene integrated into a chromosome. The co-transfection with a selectable marker such as dhfr, gpt, neomycin, zeocin or hygromycin allows the identification and isolation of the transfected cells. The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy *et al., Biochem. J. 227*: 277-279 (1991); Bebbington *et al., Biotechnology 10*: 169-175 (1992)). Using these markers, the mammalian cells are grown in selective medium and the cells with the highest resistance are selected. These cell lines contain the amplified gene(s) integrated into a chromosome. Chinese hamster ovary (CHO) are often used for the production of proteins.
The expression vector pcDNA3 contains the strong promoter (CMV) of Cytomegalovirus. Multiple cloning sites, e.g., with the restriction enzyme cleavage sites EcoRI, XhoI, facilitate the cloning of the gene of interest. The vectors contain in addition the 3' intron, the polyadenylation and termination signal of the bovine growth hormone gene.

### Example 2(a): Cloning and Expression in COS Cells

The expression plasmid is made by cloning a cDNA encoding Kir7.1 into the expression vector pcDNA3 (which can be obtained from Invitrogen, Inc.).
The expression vector pcDNA3 contains: **(**1) an *E. coli* origin of replication effective for propagation in *E. coli* and other prokaryotic cells; (2) an ampicillin resistance gene for selection of plasmid-containing prokaryotic cells; (3) an SV40 origin of replication for propagation in eukaryotic cells; (4) a CMV promoter, a polylinker; (5) a polyadenylation from the bovine growth hormone gene arranged so that a cDNA can be conveniently placed under expression control of the CMV promoter and operably linked to the polyadenylation signal by means of restriction sites in the polylinker. pcDNA3 contains, in addition, the selectable neomycin marker.
A DNA fragment encoding the Kir7.1 is cloned into the polylinker region of the vector so that recombinant protein expression is directed by the CMV promoter. The plasmid construction strategy is as follows. The Kir7.1 cDNA of the deposited clone is in the bluescript vector (Stratagene), it is excised from the bluescript vector with KpnI and Not I. The vector, pcDNA3, is digested with EcoRI and Xho I. The PCR amplified DNA fragment and the linearized vector are then ligated. The ligation mixture is transformed into *E. coli* strain DH5α (available from GIBCO BRL), and the transformed culture is plated on ampicillin media plates which then are incubated to allow growth of ampicillin resistant colonies. Plasmid DNA is isolated from resistant colonies and examined by restriction analysis or other means for the presence of the Kir7.1-encoding fragment.
For expression of recombinant Kir7.1, COS cells are transfected with an expression vector, as described above, using DEAE-Dextran, as described, for instance, in Sambrook *et al., Molecular Cloning: a Laboratory Manual*, Cold Spring Laboratory Press, Cold Spring Harbor, New York (1989). Cells are incubated under conditions for expression of Kir7.1 by the vector.

### Example 2(b): Cloning and Expression in HEK293 Cells

The vector described in example 1 is used for the expression of Kir7.1 protein. The plasmid contains the mouse neomycin resistance gene under control of the SV40 early promoter. HEK293 cells are transfected with these plasmids can be selected by growing the cells in a selective medium (containing 1 mg/ml Geneticin, Life Technologies). Cells grown in presence of 1mg/ml concentrations of Geneticin develop resistance to the drug by producing the target enzyme, Neomycin Resistance. If a second gene is linked to the Neomycin Resistance gene, it is usually co-expressed. It is known in the art that this approach may be used to develop cell lines expressing large amounts of the protein of interest, up to 1 pmole per mg of cell membrane in the case of a receptor. Subsequently, when the Geneticin is withdrawn, cell lines are obtained which contain the amplified gene integrated into one or more chromosome(s) of the host cell.
Clontech's Tet-Off and Tet-On gene expression systems and similar systems can be used to express Kir7.1 in a regulated way in mammalian cells (Gossen M. and Bujard H., *Proc. Natl*. *Acad. Sci. USA 89*: 5547-5551(1992)). For the polyadenylation of the mRNA other signals, e.g., from the human growth hormone or globin genes can be used as well. Stable cell lines carrying a gene of interest integrated into the chromosomes can also be selected upon co-transfection with a selectable marker such as gpt, G418 or hygromycin.
HEK293 cells are used for transfection. 20 µg of the expression plasmid is transfected using calcium phosphate (Chen C. and Okayama H., (1987) *Mol. Cell. Biol.*; 7 : 2745-2752). The plasmid pcDNA3 contains a dominant selectable marker, the neomycin resistance gene from Tn5 encoding an enzyme that confers resistance to a group of antibiotics including Geneticin. The cells are seeded in MEM supplemented with 1 mg/ml Geneticin. After 2 days, the cells are trypsinized and seeded in cloning plates in MEM supplemented with 1 mg/ml Geneticin. After about 10-14 days, single clones are trypsinized and then seeded in 6-well petri dishes or 10 ml flasks. Clones growing are then transferred to new 6-well plates. Expression of the desired gene product is analyzed, for instance, by Northern blot.

### Example 3

### Tissue distribution of Kir7.1 mRNA expression

Northern blot analysis can be carried out to examine Kir7.1 gene expression in human tissues, using methods described by, among others, Sambrook *et al*., cited above. A cDNA probe containing the entire nucleotide sequence of the Kir7.1 protein (Figure 1a) can be labeled with ³²P using the Rediprime™ DNA labeling system (Amersham Life Science, Arlington, IL), according to manufacturer's instructions. After labeling, the probe can be purified using a CHROMA SPIN- 100™ column (Clontech Laboratories, Inc.), according to manufacturer's protocol number PT1200-1. The purified labeled probe is then used to examine various human tissues for Kir7.1 mRNA.
Multiple Tissue Northern (MTN) blots containing various human tissues can be obtained from Clontech and examined with the labeled probe using ExpressHyb™ hybridization solution (Clontech) according to manufacturer's protocol number PT1190-1. Following hybridization and washing, the blots can be mounted and exposed to film at -70°C overnight, and films developed according to standard procedures.
It will be clear that the invention may be practiced otherwise than as particularly described in the foregoing description and examples.
Numerous modifications and variations of the present invention are possible in light of the above teachings and, therefore, are within the scope of the appended claims. The entire disclosure of all publications (including patents, patent applications, journal articles, laboratory manuals, books, or other documents) cited herein are incorporated by reference.

## Claims

1. An isolated polynucleotide selected from the group consisting of:
(a) a polynucleotide comprising a nucleotide sequence that has at least 80% identity to a nucleotide sequence encoding the Kir7.1 polypeptide of SEQ ID NO:2 over its entire length; or a nucleotide sequence complementary to said nucleotide sequence,
(b) a polynucleotide comprising a nucleotide sequence that has at least 80% identity to a nucleotide sequence encoding the Kir7.1 polypeptide expressed by the cDNA insert deposited at the ATCC with Deposit Number 209321 ; or a nucleotide sequence complementary to said nucleotide sequence.

2. A polynucleotide according to claim 1 which is DNA or RNA.

3. A polynucleotide according to one of claim 1 and 2 wherein said nucleotide sequence is at least 90% identical to that contained in SEQ ID NO:1.

4. A polynucleotide according to one of claim 1 to 3 wherein said nucleotide sequence comprises the Kir7.1 polypeptide encoding sequence contained in SEQ ID NO:1.

5. A polynucleotide according to claim 3 which is polynucleotide of SEQ ID NO: 1.

6. A DNA or RNA molecule comprising an expression system, wherein said expression system is capable of producing a Kir7.1 polypeptide comprising an amino acid sequence, which has at least 80% identity with the polypeptide of SEQ ID NO:2 when said expression system is present in a compatible host cell.

7. A host cell comprising the expression system of claim 6.

8. A process for producing a Kir7.1 polypeptide comprising culturing a host of claim 7 under conditions sufficient for the production of said polypeptide and recovering the polypeptide from the culture.

9. A process for producing a cell which produces a Kir7.1 polypeptide thereof comprising transforming or transfecting a host cell with the expression system of claim 6 such that the host cell, under appropriate culture conditions, produces a Kir7.1 polypeptide.

10. A Kir7.1 polypeptide comprising an amino acid sequence which is at least 80% identical to the amino acid sequence of SEQ ID NO:2 over its entire length.

11. The polypeptide of claim 10 which comprises the amino acid sequence of SEQ ID NO:2.

12. A Kir7.1 polypeptide comprising an amino acid sequence which is at least 80% identical to the amino acid sequence encoded by the cDNA contained in ATCC 209321

13. An antibody immunospecific for the Kir7.1 polypeptide of one of claims 10 to 12.

14. Use of an agonist of Kir7.1 polypeptide of one of claims 10 to 12 for the manufacture of a medicament for the treatment of a subject in need of enhanced activity or expression of said Kir7.1 polypeptide.

15. Use of an antagonist of Kir7.1 polypeptide of one of claims 10 to 12 for the manufacture of a medicament for the treatment of a subject having need to inhibit activity or expression of said Kir7.1 polypeptide.

16. A process for diagnosing a disease or a susceptibility to a disease in a subject related to expression or activity of Kir7.1 polypeptide of one of claims 10 to 12 in a subject comprising:
(a) determining the presence or absence of a mutation in the nucleotide sequence encoding said Kir7.1 polypeptide in the genome of said subject; and/or
(b) analyzing for the presence or amount of the Kir7.1 polypeptide expression in a sample derived from said subject.

17. A process for identifying agonists to Kir7.1 polypeptide of one of claims 10 to 12 comprising:
(a) contacting cells produced by claim 9 with a candidate compound; and
(b) determining whether the candidate compound effects a signal generated by activation of the Kir7.1 polypeptide.

18. An agonist identified by the method of claim 17.

19. A process for identifying antagonists to Kir7.1 polypeptide of one of claims 10 to 12 comprising:
(a) contacting said cell produced by claim 9 with an agonist; and
(b) determining whether the signal generated by said agonist is diminished in the presence of a candidate compound.

20. An antagonist identified by the process of claim 19.


SEQUENCE LISTING

(1) GENERAL INFORMATION:

(i) APPLICANT:
(A) NAME: SYNTHELABO
(B) STREET: 22 avenue Galilee
(C) CITY: Le Plessis-Robinson
(E) COUNTRY: FRANCE
(F) POSTAL CODE (ZIP): 92350
(G) TELEPHONE: (33) 1 45 37 55 55

(ii) TITLE OF INVENTION: inwardly rectifying potassium channel gene
and protein

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: FR 1

(2) INFORMATION FOR SEQ ID NO: 1:

(i) SEQUENCE CHARACTERISTICS:
(A) LENGTH: 2931 base pairs
(B) TYPE: nucleic acid
(C) STRANDEDNESS: double
(D) TOPOLOGY: circular

(ii) MOLECULE TYPE: cDNA to mRNA

(vi) ORIGINAL SOURCE:
(A) ORGANISM: Homo sapiens

(ix) FEATURE:
(A) NAME/KEY: CDS
(B) LOCATION:117..1199
(D) OTHER INFORMATION:/product= "cDNA encoding an
inwardly rectifying potassium channel"

(ix) FEATURE:
(A) NAME/KEY: 5'UTR
(B) LOCATION:1..116

(ix) FEATURE:
(A) NAME/KEY: 3'UTR
(B) LOCATION:1200..2931

(ix) FEATURE:
(A) NAME/KEY: mat_peptide
(B) LOCATION:117..1196
(C) IDENTIFICATION METHOD: experimental
(D) OTHER INFORMATION:/product= "inwardly rectifying
potassium channel"
/evidence= EXPERIMENTAL
/standard_name= "Kir7.1"

(xi) SEQUENCE DESCRIPTION: SEQ ID NO: 1:
   AATTCGGCAC GAGGATTAAA ATTATCTGAT CAAAAAGGCA GACTCTGTAA ATTTCCTTAA 60
   GACCTACCTT GGCATAAAGG CTGACCCAGC AAAAGAACTG AGAAATACAG CCTGAG 116
   ATG GAC AGC AGT AAT TGC AAA GTT ATT GCT CCT CTC CTA AGT CAA AGA 164
   TAC CGG AGG ATG GTC ACC AAG GAT GGC CAC AGC ACA CTT CAA ATG GAT 212
   GGC GCT CAA AGA GGT CTT GCA TAT CTT CGA GAT GCT TGG GGA ATC CTA 260
   ATG GAC ATG CGC TGG CGT TGG ATG ATG TTG GTC TTT TCT GCT TCT TTT 308
   GTT GTC CAC TGG CTT GTC TTT GCA GTG CTC TGG TAT GTT CTG GCT GAG 356
   ATG AAT GGT GAT CTG GAA CTA GAT CAT GAT GCC CCA CCT GAA AAC CAC 404
   ACT ATC TGT GTC AAG TAT ATC ACC AGT TTC ACA GCT GCA TTC TCC TTC 452
   TCC CTG GAG ACA CAA CTC ACA ATT GGT TAT GGT ACC ATG TTC CCC AGT 500
   GGT GAC TGT CCA AGT GCA ATC GCC TTA CTT GCC ATA CAA ATG CTC CTA 548
   GGC CTC ATG CTA GAG GCT TTT ATC ACA GGT GCT TTT GTG GCG AAG ATT 596
   GCC CGG CCA AAA AAT CGA GCT TTT TCA ATT CGC TTT ACT GAC ATA GCA 644
   GTA GTA GCT CAC ATG GAT GGC AAA CCT AAT CTT ATC TTC CAA GTG GCC 692
   AAC ACC CGA CCT AGC CCT CTA ACC AGT GTC CGG GTC TCA GCT GTA CTC 740
   TAT CAG GAA AGA GAA AAT GGC AAA CTC TAC CAG ACC AGT GTG GAT TTC 788
   CAC CTT GAT GGC ATC AGT TCT GAC GAA TGT CCA TTC TTC ATC TTT CCA 836
   CTA ACG TAC TAT CAC TCC ATT ACA CCA TCA AGT CCT CTG GCT ACT CTG 884
   CTC CAG CAT GAA AAT CCT TCT CAC TTT GAA TTA GTT GTA TTC CTT TCA 932
   GCA ATG CAG GAG GGC ACT GGA GAA ATA TGC CAA AGG AGG ACA TCC TAC 980
   CTA CCG TCT GAA ATC ATG TTA CAT CAC TGT TTT GCA TCT CTG TTG ACC 1028
   CGA GGT TCC AAA GGT GAA TAT CAA ATC AAG ATG GAG AAT TTT GAC AAG 1076
   ACT GTC CCT GAA TTT CCA ACT CCT CTG GTT TCT AAA AGC CCA AAC AGG 1124
   ACT GAC CTG GAT ATC CAC ATC AAT GGA CAA AGC ATT GAC AAT TTT CAG 1172
   ATC TCT GAA ACA GGA CTG ACA GAA TAA GACTTATCCA TTTTTTAATG 1219
   TATTAAATAC ACCCAGCCAG TTATGCAGCT ACTTTTTCTT TACTGTATCT CATGTTTTCT 1279
   TTTTTCAATG CTAATTATAG CTCTCTACAT CACGGTAATC ATGCCTATGC CTACATAAGA 1339
   ATGGCTGAGC TAACAATACA CATTCTGGAA ACATAACACT CTACATTACA AAGTTTGTTA 1399
   CCTGCTGAAA TCAATGTAAC TCAACTTGAC AGACACTTAT ACAGAAATGT TGCTGGTGAA 1459
   TTTATAAGAA TGTGGTATGA TACTAGTAAT GAAGGCAAAA TGGACAGTGA AGTTTAACAC 1519
   AACTGAACTC TAAGAAAATC AACCATTAAT CTCTCATTTT CATCTGCAAA TTGAAGCAAC 1579
   AGTTTAGTTT CAAACCTAGC TCCCTGGGTG GAATGACGAC TTCACTATAC TTAGTGAATA 1639
   TCCTTTAAGA GCTGGGATTT TTTTCAAGAC AACAAAGATC ATTCATTTGG TTCTTTATAC 1699
   TATGAAACTT GAGTAAGTAT TACCTCCTTA ATTTTTAACA ACTAAGAACA AAAATTAACG 1759
   AGAAAAACAA CAAAGTACAG ATTTATACAT AAACCTAAAA GCATTTGAAC ATGACACCCG 1819
   AACACATACA TATATGTTCA CTTATTTGTG GCAGAAGGTG ATCAGATAAG CTCCAGCCCA 1879
   AATGGAACCT GTGGGGTGGT ATTTTGCATT GCAAGGAGAC GCAAAATTTT ATTTTAAAAC 1939
   TGTCCTCCAT AATAATCAAA CGGTGATTCA TCTAAATGAC TTCTAGCAAC CTAAGTAAAA 1999
   ACATTCCCCT CCTATGTATG ATTCATTTGA TCATATAAAA CATCATGATG GCTCTAATTC 2059
   ATAAATACAA AAATATATTT AAGTCTTTAT AGATATAAAG CTTTACTTAG ATATAACTTG 2119
   AGTGAGTAGG GAAAAAAATC TACAGTAGAT AAAGCAAAAG ATAATTAGGC AACAAAGCAT 2179
   TTTCAAACTC AAATTCCTGT TTCCAACTTC AAATAGTTTT TTCTATAAAC ACAAAATCAG 2239
   TGTTTATTCA CCAGTAGGAG GTTGGACTAG ATGAACTCTA TTATTTCTTT CTAAATCTAA 2299
   TAGTCTATAA AAATTATGTT TCCTCTGTTT TTTATTTTAT CTATGCTAAA ATGAGCCCTT 2359
   TCCCTTATGT CCAGTTTAAG ATGATCATTT GCATGATTTT CATTTCAATA AAAAAAAGAG 2419
   AAACTGTCCT TAAAACAAAA CAAAAACCAA AAAAGTCACC CTATCAGGTT TCAAACAGAT 2479
   TTGTGGCTGT TCTTTTCTGA AATTTCCCTT ATTCAGGTTT CTGTGGGAAA AATGAAAGAT 2539
   TAACCTTCCC CACTGGTGAT GACCTAGGCA GGAATCATCT CTTGAAATAA ATACTAGCTG 2599
   AGTAAAGGCA AGCAGGTGTG AAGAGCAGGG CTCAGCAGCA AGTCACATTT TTCTACTATT 2659
   TGACCAAAAG GAAAAGAAAA TAAAGAAGAA CTCTGGAGTG GTCTAAGACT GATAATAGCA 2719
   GAAGAATATC AAGAACACAG AAACTTAATT ATTGTGAACT TTTGCTGTTT GAAAATCTTA 2779
   GACATTCATT CTTAAGTAGA AATCAGACCA ACAGATTTTC CCAACCCAAG ACTATTGTAA 2839
   CACATAAAGA CAGCAAGAAT TCTTATTTCT ATAATAAATT AACAAGATTC ACCTAACCTT 2899
   TGAAAATAAA GTAGTATTGA AGACTTAAAA AA 2931

(2) INFORMATION FOR SEQ ID NO: 2:

(i) SEQUENCE CHARACTERISTICS:
(A) LENGTH: 361 amino acids
(B) TYPE: amino acid
(D) TOPOLOGY: linear

(ii) MOLECULE TYPE: protein
(xi) SEQUENCE DESCRIPTION: SEQ ID NO: 2: